# EUROPEAN PATENT APPLICATION

(11) **EP 2 108 358 A1**
(43) Date of publication of application: **14.10.2009**
(21) Application number: 06833232.9
(22) Date of filing: 24.11.2006
(51) Int. Cl.: A61K 8/36, A61K 8/39, A61K 8/41, A61K 8/81, A61K 8/92, A61Q 1/10

(54) **COSMETIC FOR EYELASHES**

(71) Applicant: PIAS CORPORATION, Kita-ku, Osaka-shi Osaka, 5310072 (JP)
(72) Inventor: HAMADA, Kazuhiko, Kobe-shi Hyogo 651-2241 (JP); FUJIWARA, Shigehisa, Kobe-shi Hyogo 651-2241 (JP); SHIGA, Kazuhiro, Kobe-shi Hyogo 651-2241 (JP)
(74) Representative: Stolmár, Matthias
(86) International application number: PCT/JP2006/323428
(87) International publication number: WO 2008/062530

(57) **Abstract**

It is an object of the present invention to provide a cosmetic preparation for eyelashes used as mascara and the like, which is excellent in long lash effect by improving adhesivity to eyelashes and adhesivity of pigment or fiber. The cosmetic preparation for eyelashes includes: an alkyl acrylate-vinyl acetate copolymer as an aqueous coating agent; a water-soluble polymer; a fatty acid; an alkali agent; a nonionic surfactant; and an oil-phase forming agent including beeswax.

## Description

### Field of the Invention

The present invention relates to a cosmetic preparation for eyelashes used as mascara and the like.

### Background Art

There have been generally used a cosmetic preparation for eyelashes that provides a makeup effect of highlighting eyes by giving eyelashes the appearance of being longer (long lash effect which is also referred to as '"engthening effect"), as well as serving a conventional function of of upwardly curling eyelashes. Also, in order to further improve the lengthening effect of giving eyelashes the appearance of being longer, a fiber-containing cosmetic preparation for eyelashes with natural fiber or synthetic fiber blended therein has also been used.

Conventionally, as a patent application relating to such cosmetic preparation for eyelashes, patent applications such as the following Patent Documents 1 to 3 have been filed. The invention described in Patent Document 1 relates to a cosmetic preparation for eyelashes with a cysteine, a dextrin fatty acid ester or the like content, as well as a solid oil content or volatile oil content, which is contrived in consideration of and to solve the problem in that although increase in the amount of solid oil content contained in a cosmetic preparation for eye lashes can provide a makeup effect of giving eyelashes the appearance of being thicker, the cosmetic preparation is hardened and that results in an uncomfortableness in use, while a large amount of coating agent blended therein results in a respective eyelash to be sticked with each other or an uncomfortableness in use.

The invention described in Patent Document 2 relates to a cosmetic preparation with a hydrogenated styrene methylstyrene indene copolymer or trimethyl siloxy silicate content, which is contrived in consideration of and to solve the problem in that most of the conventional cosmetic preparation for eyelashes are an oil-in-water type emulsion with wax or coat-forming resin dispersed in water, however, cosmetic preparation of such oil-in-water type does not provide a satisfactory makeup effect of curling up the eyelashes for highlighting eyes, while excessively large amount of wax blended therein results in drying, fear in causing an uncomfortableness in use, or the like.

The invention described in Patent Document 3 relates to a fiber-containing cosmetic preparation for eyelashes that uses modified cross-section hollow-fiber in considerartion of the problem in that a sufficient long lash effect cannot be achieved by simply blending fiber.

However, the conventional cosmetic preparation for eyelashes had problems in that adhesivity to eyelashes or to pigment is not necessarily sufficient, and an effect such as lengthening effect is not necessarily sufficient. Such problems could not have been solved yet even by the inventions described in the Patent Documents 1 to 3. Particularly, in the fiber-cosmetic preparation for eyelashes, the insufficient adhesivity of a coat-forming agent in the cosmetic preparation might incur detachment of the fiber from the eyelashes. Therefore, there has been a demand to solve the above problems.

Patent Document 1: Japanese Patent Application Laid-Open No. 2003-335633
Patent Document 2: Japanese Patent Application Laid-Open No. 2005-247730
Patent Document 3: Japanese Patent Application Laid-Open No. 2006-273864

### Disclosure of the Invention

### Problems that the Invention is to solve

The present invention has been contrived to solve the above-mentioned problems, and it is an object of the present invention to provide a cosmetic preparation for eyelashes which is excellent in long lash effect by improving adhesivity to eyelashes and adhesivity of pigment or fiber.

### Means for Solving the Problems

The present invention is contrived to solve the above-mentioned problems and there is provided a cosmetic preparation for eyelashes that contains an alkyl acrylate-vinyl acetate copolymer as an aqueous coating agent; a water-soluble polymer; a fatty acid; an alkali agent; a nonionic surfactant; and an oil-phase forming agent including beeswax.

### Advantage of the Invention

As described above, since the cosmetic preparation for eyelashes of the present invention contains an alkyl acrylate-vinyl acetate copolymer as an aqueous coating agent, a water-soluble polymer, a fatty acid; an alkali agent, a nonionic surfactant, and oil-phase forming agent including beeswax, it enables itself to be excellent in long lash effect by improving adhesivity to eyelashes and adhesivity of fiber.

### Best mode for Carrying Out the Invention

Now, the description will be made for an embodiment of the present invention.
As described above, the cosmetic preparation for eyelashes of the present invention contains the alkyl acrylate-vinyl acetate copolymer as the aqueous coating agent; the water-soluble polymer; the fatty acid; the alkali agent; the nonionic surfactant; and the oil-phase forming agent including beeswax.

The aqueous coating agent is a component contained in the cosmetic preparation for eyelashes which is applied on the eyelashes so as to be capable of forming a coat thereon, and has characteristics of being able to be dissolved or dispersed in water. In the present invention, the alkyl acrylate-vinyl acetate copolymer is used as the aqueous coating agent. A content of the aqueous coating agent is preferably in the range of 3 % by weight to 40 % by weight. By giving the amount of 3 % by weight or more of the agent, a favorable fixing property to the eyelashes can be given to the cosmetic preparation. By giving the amount of 40 % by weight or less of the agent, a favorable coating property can be obtained. From this point of view, it is more preferable to give 5 % by weight to 25 % by weight of the agent.

As the water-soluble polymer, a polyvinyl pyrrolidone is mainly used. Other than the polyvinyl pyrrolidone, for example, hydroxyl ethyl cellulose or the like may also be used. Polyvinyl pyrrolidone and hydroxyl ethyl cellulose may be contained in the cosmetic preparation for eyelashes in combination thereof or alone.

As the fatty acid, a stearic acid is mainly used. Other than the stearic acid, for example, a lauric acid, a myristic acid, a palmitic acid or the like may also be used. As the alkali agent, 2-amino-2-methyl-1-prophanol is mainly used. Other than 2-amino-2-methyl-1-prophanol, triethanolamine, sodium hydroxide, potassium hydroxide or the like may also be used.

As the nonionic surfactant, for example, sorbitan monostearate, sorbitan polyoxyethylene monostearate (20) or the like is used. These may be used in combination thereof or alone. In addition, nonionic surfactants other than the above may be used.

The oil-phase forming agent is a component that forms an oil-phase in the cosmetic preparation for eyelashes. In the present invention, beeswax, carnauba wax, vaseline, liquid paraffin or the like is used as oil-phase forming agent. The amount of oil-phase forming agent contained in the cosmetic preparation is preferably in the range of 3 % by weight to 30 % by weight. By giving the amount of 3 % by weight or more of the agent, a favorable fixing property to the eyelashes can be given to the cosmetic preparation. By giving the amount of 30 % by weight or less of the agent, a favorable coating property can be obtained. From this point of view, it is more preferable to give 5 % by weight to 20 % by weight of the agent, and is further preferable to give 10 % by weight to 15 % by weight of the agent.

In the present invention, among the components of the oil-phase forming agent, the beeswax is an essential component of the oil-phase forming agent. With the beeswax contained in the oil-phase forming agent, a remarkably excellent adhesivity to the eyelashes can be given to the cosmetic preparation. Although the content of the beeswax relative to the total weight of the oil-phase forming agent is not particularly limited, it is preferably in the range of 50 % by weight to 100 % by weight. By giving the amount of 50 % by weight or more, an appropriate adhesivity of the cosmetic preparation to the eyelashes can be obtained.

In the cosmetic preparation for eyelashes of the present invention, fiber or coloring agent is further contained. As the fiber, each natural fiber and synthetic fiber may be used. As the natural fiber, for example, cotton, silk, wool, keratin fiber or the like may be used. As the synthetic fiber, polyester, polyamide, polyethylene, polypropylene or the like may be used. In addition, artificial fiber such as rayon may also be used.

Although the content of the fiber is not particularly limited, the fiber may be contained in the range of 0.5 % by weight to 10 % by weight relative to the total weight. Furthermore, although the length of the fiber is not limited, it may be in the range of 0.5 mm to 4.0 mm.

As the coloring agent, pigment or dye may be used. As the pigment, both inorganic pigment and organic pigment may be used. An arbitrary pigment, such as iron oxide, titanium dioxide, zirconium oxide, zinc oxide, cerium oxide, chromium oxide, carbon black, mica or the like may be used. As the dye, arbitrary dye, such as Yellow No. 4, Yellow No. 403 (1), Red No. 201, Red No. 227, Blue No. 1, and Black No. 401 or the like may be used. Although the blending amount of the coloring agent is not particularly limited, as for the pigment, for example, it may be blended in the range of 0.5 % by weight to 15 % by weight with respect to the total weight, and as for the dye, it may be blended in the range of 0.00001 % by weight to 1 % by weight with respect to the total weight.

Furthermore, in the cosmetic preparation for eyelashes of the present invention, in addition to the components described above, a component which may be appropriately blended in the cosmetic preparation for makeup may be added in a quantitative or qualitative range that is not violating the effect of the invention. For example, antiseptics, alcohols, polyhydric alcohols, drugs, thickeners, clay minerals, fragrances, antioxidants, ultraviolet absorbing agents, humectants, hydrocarbon oils may be optionally blended.

### EXAMPLES

### (Example 1)

The blending components and blending amount of the cosmetic preparation for eyelashes in the present example are as follows.

| Component | Bending amount (% by weight) |
|---|---|
| Alkyl acrylate-vinyl acetate copolymer | 8.0 % |
| Polyvinyl pyrrolidone | 1.0 % |
| Sorbitan monostearate | 0.5 % |
| Stearic acid | 2.0 % |
| 2-amino-2-metyl-1-propanol | 0.6 % |
| Beeswax | 8.0 % |
| Candelilla wax | 2.0 % |
| Propylene glycol | 5.0 % |
| Black iron oxide | 8.0 % |
| Methyl paraben | 0.1 % |
| Ion-exchanged water | remainder |

A preparation process is described below. The cosmetic preparation for eyelashes of the Example 1 was prepared as follows:
(A) firstly, heating and dissolving beeswax, candelilla wax, stearic acid, and sorbitan monostearate to the temperature between 85 to 90°C;
(B) secondly, heating and dissolving, or dispersing ion-exchanged water, methyl paraben, propylene glycol, 2-amino-2-methyl-1-propanol, and pigment (black iron oxide) to the temperature between 85 to 90°C;
(C) thirdly, mixing (A) and (B), dispersing the mixture with the use of a homomixer, and then cooling by agitating to 40°C;
(D) fourthly, adding polyvinyl pyrrolidone and the alkyl acrylate-vinyl acetate copolymer which are peliminarily disoluted in water to (C); and
(E) mixing the (D) until it becomes homogeneous.

### (Example 2)

The blending components and blending amounts of the cosmetic preparation for eyelashes in a present example are as follows.

| Component | Blending amount (% by weight) |
|---|---|
| Alkyl acrylate-vinyl acetate copolymer | 8.0 % |
| Polyvinyl pyrrolidone | 1.0 % |
| Sorbitan monostearate | 0.5 % |
| Stearic acid | 2.0 % |
| Triethanolamine | 0.4 % |
| Beeswax | 8.0 % |
| Candelilla wax | 2.0 % |
| Propylene glycol | 5.0 % |
| Black iron oxide | 8.0 % |
| Methyl paraben | 0.1 % |
| Ion-exchanged water | remainder |

In the present example, as the alkali agent, triethanolamine was used instead of 2-amino-2-metyl-1-propanol used in the Example 1. Other blending components and blending amounts are same as the Example 1. A preparation process was same as the Example 1.

### (Example 3)

The blending components and blending amounts of the cosmetic preparation for eyelashes of a present example are as follows.

| Component | Blending amount (% by weight) |
|---|---|
| Alkyl acrylate-vinyl acetate copolymer | 8.0 % |
| Polyvinyl pyrrolidone | 1.0 % |
| Sorbitan monostearate | 0.5 % |
| Stearic acid | 2.0 % |
| Sodium hydroxide | 0.2 % |
| Beeswax | 8.0 % |
| Candelilla wax | 2.0 % |
| Propylene glycol | 5.0 % |
| Black iron oxide | 8.0 % |
| Methyl paraben | 0.1 % |
| Ion-exchanged water | remainder |

In the present example, as the alkali agent, sodium hydroxide was used instead of 2-amino-2-metyl-1-propanol in the Example 1. Other blending components and blending amounts are same as the Example 1. A preparation process was same as the Example 1.

### (Comparative Example 1)

As Comparative Example 1, the following blending components of the following blending amounts were prepared. In the Comparative Example 1, polyvinyl pyrrolidone which is the water-soluble polymer was not blended. Other blending components and blending amounts were same as the Example 1. A preparation process was same as the Example 1.

| Component | Blending amount (% by weight) |
|---|---|
| Alkyl acrylate-vinyl acetate copolymer | 8.0 % |
| Sorbitan monostearate | 0.5 % |
| Stearic acid | 2.0 % |
| 2-amino-2-metyl-1-propanol | 0.6 % |
| Beeswax | 8.0 % |
| Candelilla wax | 2.0 % |
| Propylene glycol | 5.0 % |
| Black iron oxide | 8.0 % |
| Methyl paraben | 0.1 % |
| Ion-exchanged water | remainder |

### (Test Example 1)

In respect to Examples 1 to 3 and Comparative Example 1, each test for adhesivity to eyelashes, adhesivity of the pigment, and makeup effect (lengthening property) was carried out. Here, the lengthening property means the makeup effect of giving eyelashes the appearance of being longer. Each test for the adhesivity to eyelashes, the adhesivity of the pigment, and the makeup effect (lengthening property) was carried out on the basis of an evaluation (5 steps of evaluation) of 10 monitors. The test results are shown in Table 1.

In table 1, ⊚ represents very good, ○ represents good, and Δ represents neither of them. Although the other evaluation steps of bad and very bad were also provided besides the above, there was no relevant answer in the present test.

**[Table 1]**

| | Example 1 | Example 2 | Example 3 | Comparative Example 1 |
|---|---|---|---|---|
| Adhesivity to Eyelashes | ⊚ | ○ | ○ | △ |
| Adhesivity of Pigment | ⊚ | ⊚ | ⊚ | △ |
| Lengthening property | ⊚ | ○ | ○ | △ |

As is clear from the Table 1, the adhesivity to eyelashes, the adhesivity of the pigment, and the makeup effect (lengthening property) all showed very favorable results of ⊚ in Example 1 containing 2-amino-2-methyl-1-propanol as the alkali agent, in addition to the alkyl acrylate-vinyl acetate copolymer as the aqueous coating agent, polyvinyl pyrrolidone as the water-soluble polymer, sorbitan monostearate as the nonionic surfactant, the stearic acid as the fatty acid, and beeswax and candelilla wax that each constitute the oil-phase forming agent.

Even though it was not as favorable as the Example 1, favorable results of ⊚ for the adhesivity of pigment and ○ for the adhesivity to eyelashes and the lengthening effect were obtained in Example 2 in which triethanolamine is used instead of 2-amino-2-methyl-1-propanol as an alkali agent and Example 3, in which sodium hydroxide is used instead of 2-amino-2-methyl-1-propanol as an alkali agent.

On the other hand, the adhesivity of the pigment, the adhesivity to eyelashes, and the lengthening property showed all Δ in the Comparative Example 1 in which polyvinyl pyrrolidone which is the water-soluble polymer is not contained.

From this test result, it is assumable that with the alkali agent blended, a neutralization reaction with fatty acid takes place, which brings the alkyl acrylate-vinyl acetate copolymer which is the aqueous coating agent to be appropriately dispersed.

Even with the presence of the alkali agent, favorable results were not obtained in the Comparative Example 1 in which polyvinyl pyrrolidone which is the water-soluble polymer was not blended. As a result, it is considered that polyvinyl pyrrolidone contributes to the adhesivity of the pigment, the adhesivity to eyelashes, and the lengthening property.

Particularly, since the most favorable results were obtained by using 2-amino-2-methyl-1-propanol as the alkali agent, it is admissible that the adhesivity to eyelashes and the lengthening property are improved by an interaction between polyvinyl pyrrolidone and 2-amino-2-methyl-1-propanol.

### (Example 4)

The blending components and blending amounts of the cosmetic preparation for eyelashes of a present example are as follows. A preparation method was same as Example 1.

| Component | Blending amount (% by weight) |
|---|---|
| Alkyl acrylate-vinyl acetate copolymer | 10.0 % |
| Polyvinyl pyrrolidone | 1.0 % |
| Sorbitan monostearate | 0.5 % |
| Stearic acid | 2.0 % |
| 2-amino-2-metyl-1-propanol | 0.6 % |
| Beeswax | 8.0 % |
| Candelilla wax | 2.0 % |
| Propylene glycol | 5.0 % |
| Black iron oxide | 8.0 % |
| Methyl paraben | 0.1 % |
| Ion-exchanged water | remainder |

### (Example 5)

The blending components and blending amounts of the cosmetic preparation for eyelashes of a present example are as follows. Comparing to Example 4, the blending amount of sorbitan monostearate was doubled while the blending amount of stearic acid and 2-amino-2-metyl-1-propanol was halved. Other components and blending amounts are same as the Example 4. A preparation method was same as Example 1.

| Component | Blending amount (% by weight) |
|---|---|
| Alkyl acrylate-vinyl acetate copolymer | 10.0 % |
| Polyvinyl pyrrolidone | 1.0 % |
| Sorbitan monostearate | 1.0 % |
| Stearic acid | 1.0 % |
| 2-amino-2-metyl-1-propanol | 0.3 % |
| Beeswax | 8.0 % |
| Candelilla wax | 2.0 % |
| Propylene glycol | 5.0 % |
| Black iron oxide | 8.0 % |
| Methyl paraben | 0.1 % |
| Ion-exchanged water | remainder |

### (Comparative Example 2)

As Comparative Example 2, the following blending components of the following blending amounts were prepared. Except that sorbitan monostearate was not blended, the components and blending amounts were same as the Example 4. A preparation method was same as Example 1.

| Component | Blending amount (% by weight) |
|---|---|
| Alkyl acrylate-vinyl acetate copolymer | 10.0 % |
| Polyvinyl pyrrolidone | 1.0 % |
| Stearic acid | 2.0 % |
| 2-amino-2-metyl-1-propanol | 0.6 % |
| Beeswax | 8.0 % |
| Candelilla wax | 2.0 % |
| Propylene glycol | 5.0 % |
| Black iron oxide | 8.0 % |
| Methyl paraben | 0.1 % |
| Ion-exchanged water | remainder |

### (Test Example 2)

In respect to Examples 4, 5 and Comparative Example 2, each test for the adhesivity to eyelashes, the adhesivity of the pigment, and the makeup effect (lengthening property) was carried out. The test results are shown in Table 2.

**[Table 2]**

| | Example 4 | Example 5 | Comparative Example 2 |
|---|---|---|---|
| Adhesivity to Eyelashes | ⊚ | ○ | △ |
| Adhesivity of Pigment | ⊚ | ⊚ | ⊚ |
| Lengthening property | ⊚ | △ | △ |

As is clear from the Table 2, the adhesivity to eyelashes, the adhesivity of pigment, and the makeup effect (lengthening property) all showed very favorable results of ⊚ in Example 4 containing sorbitan monostearate as the nonionic surfactant, in addition to the alkyl acrylate-vinyl acetate copolymer as the aqueous coating agent, polyvinyl pyrrolidone as the water-soluble polymer, beeswax and candelilla wax that each constitute the oil-phase forming agent, stearic acid as the fatty acid, and 2-amino-2-methyl-1-propanol as the alkali agent. In Example 5, in which the blending amount of the stearic acid and 2-amino-2-methyl-1-propanol was halved, the lengthening property was Δ. However, Example 5 as a whole showed relatively favorable result in which the adhesivity of the pigment showed the result of ⊚ and the adhesivity to eyelashes showed the result of ○.

On the other hand, in Comparative Example 2 in which sorbitan monostearate which is the nonionic surfactant was not blended, although the adhesivity of the pigment showed ⊚, both adhesivity to eyelashes and the lengthening property showed Δ.

From the above test result, it is admissible that the adhesivity of the pigment, the adhesivity to eyelashes, and the lengthening property are improved not only by the interaction between the alkali agent with the fatty acid, but also by an interaction between these with sorbitan monostearate which is the nonionic surfactant.

### (Example 6)

The blending components and blending amounts of the cosmetic preparation for eyelashes of a present example are as follows. A preparation method was same as Example 1.

| Component | Blending amount (% by weight) |
|---|---|
| Alkyl acrylate-vinyl acetate copolymer | 10.0 % |
| Polyvinyl pyrrolidone | 1.0 % |
| Hydroxyl ethyl cellulose | 0.5 % |
| Sorbitan monostearate | 0.5 % |
| Stearic acid | 2.0 % |
| 2-amino-2-metyl-1-propanol | 0.6 % |
| Beeswax | 10.0 % |
| Propylene glycol | 5.0 % |
| Hectorite | 1.0 % |
| Black iron oxide | 8.0 % |
| Polyamide fiber | 2.0 % |
| Methyl paraben | 0.1 % |
| Ion-exchanged water | remainder |

### (Comparative Example 3)

As Comparative Example 3, the following blending components of the following blending amounts were prepared. As the aqueous coating agent, the alkyl acrylate copolymer was used instead of the alkyl acrylate-vinyl acetate copolymer in the Example 6. Other blending components and blending amounts were same as the Example 6. A preparation process was same as Example 1.

| Component | Blending amount (% by weight) |
|---|---|
| Alkyl acrylate-copolymer | 10.0 % |
| Polyvinyl pyrrolidone | 1.0 % |
| Hydroxyl ethyl cellulose | 0.5 % |
| Sorbitan monostearate | 0.5 % |
| Stearic acid | 2.0 % |
| 2-amino-2-metyl-1-propanol | 0.6 % |
| Beeswax | 10.0 % |
| Propylene glycol | 5.0 % |
| Hectorite | 1.0 % |
| Black iron oxide | 8.0 % |
| Polyamide fiber | 2.0 % |
| Methyl paraben | 0.1 % |
| Ion-exchanged water | remainder |

### (Comparative Example 4)

As Comparative Example 4, the following blending components of the following blending amounts were prepared. As the aqueous coating agent, the alkyl acrylate-vinyl acetate copolymer was used. However, polyvinyl pyrrolidone and hydroxyl cellulose which are water-soluble polymers were not blended. Other blending components and blending amounts were same as the Example 6. A preparation method was same as Example 1.

| Component | Blending amount (% by weight) |
|---|---|
| Alkyl acrylate-vinyl acetate copolymer | 10.0 % |
| Sorbitan monostearate | 0.5 % |
| Stearic acid | 2.0 % |
| 2-amino-2-metyl-1-propanol | 0.6 % |
| Beeswax | 12.0 % |
| Propylene glycol | 5.0 % |
| Hectorite | 1.0 % |
| Black iron oxide | 8.0 % |
| Polyamide fiber | 2.0 % |
| Methyl paraben | 0.1 % |
| Ion-exchanged water | remainder |

### (Test Example 3)

In respect to Example 6 and Comparative Examples 3 and 4, each test for the adhesivity to eyelashes, the adhesivity of the pigment and fiber, and the makeup effect (lengthening property) was carried out. The test results are shown in Table 3.

**[Table 3]**

| | Example 6 | Comparative Example 3 | Comparative Example 4 |
|---|---|---|---|
| Adhesivity to Eyelashes | ⊚ | ○ | △ |
| Adhesion of Pigment and Fiber | ⊚ | ○ | ○ |
| Lengthening effect | ⊚ | △ | △ |

As is clear from the Table 3, the adhesivity to eyelashes, the adhesivity of pigment and fiber, and makeup effect (lengthening property) all showed very favorable results of ⊚ in Example 6 containing the alkyl acrylate-vinyl acetate copolymer as the aqueous coating agent, and polyvinyl pyrrolidone and hydroxyl ethyl cellulose as the water-soluble polymer, in addition to beeswax as the oil-phase forming agent, the stearic acid as the fatty acid, 2-amino-2-methyl-1-propanol as the alkali agent, and sorbitan monostearate as the nonionic surfactant.

On the other hand, in Comparative Example 3 using the alkyl acrylate copolymer as the aqueous coating agent instead of the alkyl acrylate-vinyl acetate copolymer, the adhesivity to eyelashes and the adhesivity of the pigment and fiber showed ○ but the lengthening property showed Δ. The favorable results were not necessarily obtained.

In addition, in Comparative Example 4 not containing the water-soluble polymer such as polyvinyl pyrrolidone or hydroxyl ethyl cellulose, the adhesivity of pigment and fiber showed ○ but both the adhesivity to eyelashes and the lengthening property showed Δ. The favorable results were not necessarily obtained.

From this test result, it is admissible that the adhesivity of the pigment, the adhesivity to eyelashes, and the lengthening property are improved by the interaction between the alkyl acrylate-vinyl acetate copolymer which is the aqueous coating agent, with the water-soluble polymer. On the other hand, when alkyl acrylate copolymer, even though the aqueous coating agent which is relatively similar to the alkyl acrylate-vinyl acetate copolymer, is used, it has been found that the interaction with the water-soluble polymer such as polyvinyl pyrrolidone or hydroxyl ethyl cellulose is not necessarily working.

### (Example 7)

The blending components and blending amounts of the cosmetic preparation for eyelashes of a present example are as follows. A preparation method was same as Example 1.

| Component | Blending amount (% by weight) |
|---|---|
| Alkyl acrylate-vinyl acetate copolymer | 10.0 % |
| Polyvinyl Pyrrolidone | 1.0 % |
| Sorbitan monostearate | 0.5 % |
| Stearic acid | 2.0 % |
| 2-amino-2-metyl-1-propanol | 0.6 % |
| Beeswax | 10.0 % |
| Vaseline | 0.4 % |
| Fluid paraffin | 0.4 % |
| Propylene glycol | 5.0 % |
| Hectorite | 1.0 % |
| Black iron oxide | 8.0 % |
| Polyamide fiber | 2.0 % |
| Methyl paraben | 0.1 % |
| Ion-exchanged water | remainder |

### (Comparative Example 5)

As Comparative Example 5, the following blending components of the following blending amounts were prepared. Candelilla wax was used instead of beeswax of Example 7, as a component of the the oil-phase forming agent. The amount of vaseline or fluid paraffin as another component of the oil-phase forming agent was increased as compared to Example 7. A preparation method was same as Example 1.

| Component | Blending amount (% by weight) |
|---|---|
| Alkyl acrylate-vinyl acetate copolymer | 10.0 % |
| Polyvinyl pyrrolidone | 1.0 % |
| Sorbitan monostearate | 0.5 % |
| Stearic acid | 2.0 % |
| 2-amino-2-metyl-1-propanol | 0.6 % |
| Candelilla wax | 8.0 % |
| Vaseline | 2.0 % |
| Fluid paraffin | 2.0 % |
| Propylene glycol | 5.0 % |
| Hectorite | 1.0 % |
| Black iron oxide | 8.0 % |
| Polyamide fiber | 2.0 % |
| Methyl paraben | 0.1 % |
| Ion-exchanged water | remainder |

### (Test Example 4)

In respect to Example 7 and Comparative Example 5, each test for the adhesivity to eyelashes, the adhesivity of the pigment and fiber, and the makeup effect (lengthening property) was carried out. The test results are shown in Table 4.

**[Table 4]**

| | Example 7 | Comparative Example 5 |
|---|---|---|
| Adhesivity to Eyelashes | ⊚ | △ |
| Adhesivity of Pigment | ⊚ | ○ |
| and Fiber Lengthening Property | ⊚ | △ |

As is clear from the Table 4, the adhesivity to eyelashes, the adhesivity of the pigment and the fiber, and the makeup effect (lengthening property) showed very favorable results of ⊚ in Example 7 using beeswax as a component of the oil-phase forming agent.

On the other hand, in Comparative Example 5 using candelilla wax as a component of the oil-phase forming agent, the adhesivity of the pigment and fiber showed ○ but the adhesivity to eyelashes and the lengthening property showed Δ. The favorable results were not necessarily obtained.

From this test result, it is found that the use of beeswax as the oil-phase forming agent is necessary to improve the adhesivity to eyelashes, the adhesivity of the pigment and the fiber, and the makeup effect (lengthening property).

## Claims

1. A cosmetic preparation for eyelashes, comprising:
an alkyl acrylate-vinyl acetate copolymer as an aqueous coating agent; a water-soluble polymer; a fatty acid; an alkali agent; a nonionic surfactant; and an oil-phase forming agent including beeswax.

2. The cosmetic preparation for eyelashes according to Claim 1, wherein the water-soluble polymer is polyvinyl pyrrolidone.

3. The cosmetic preparation for eyelashes according to Claim 1, wherein the fatty acid is a stearic acid.

4. The cosmetic preparation for eyelashes according to Claim 1, wherein the alkali agent is 2-amino-2-methyl-1-propanol.

5. The cosmetic preparation for eyelashes according to Claim 1, wherein the nonionic surfactant is at least any one of sorbitan monostearate and sorbitan polyoxyethylene monostearate (20).

6. The cosmetic preparation for eyelashes according to Claim 1, wherein the content of the aqueous coating agent is in the range of 3 % by weight to 40 % by weight.

7. The cosmetic preparation for eyelashes according to Claim 1, wherein the content of the aqueous coating agent is in the range of 5 % by weight to 25 % by weight.

8. The cosmetic preparation for eyelashes according to Claim 1, wherein the content of the oil-phase forming agent is in the range of 3 % by weight to 30 % by weight.

9. The cosmetic preparation for eyelashes according to Claim 1, wherein the content of the oil-phase forming agent is in the range of 10 % by weight to 15 % by weight.

10. The cosmetic preparation for eyelashes according to Claim 1, wherein the oil-phase forming agent is composed of beeswax, carnauba wax, vaseline, and fluid paraffin.

11. The cosmetic preparation for eyelashes according to Claim 10, wherein the content of the beeswax with respect to the total weight of oil-phase forming agent is in the range of 50 to 100 % by weight.

12. The cosmetic preparation for eyelashes according to Claim 1, further comprising fiber and a coloring agent.
